# FASCICULE DE BREVET EUROPEEN

(11) **EP 1 004 592 B1**
(45) Date de publication et mention de la délivrance du brevet: **16.07.2003**
(21) Numéro de dépôt: 99402907.2
(22) Date de dépôt: 23.11.1999
(51) Int. Cl.: C07H 17/08, A61K 31/70, C07D 231/12, C07D 401/04

(54) **Nouveaux dérivés de l'érythromycine, leur procédé de préparation et leur application comme médicaments**
Derivate von Erythromycin, ihr Verfahren zur Herstellung, und ihre Verwendung als Arzneimittel
Derivatives of erythromycin, their process of preparation and their application as medicaments

(30) Priorité: 24.11.1998 FR 9814782
(43) Date de publication de la demande: 31.05.2000
(73) Titulaire: Aventis Pharma S.A., 92160 Antony (FR)
(72) Inventeur: Denis, Alexis, 75011 Paris (FR)

(56) Documents cités:
- EP-A- 0 596 802
- EP-A- 0 799 833
- WO-A-98/56800
- DE-A- 2 756 852
- GB-A- 2 288 174
- R.FERRONI ET AL.: "Synthesis and Platelet Aggregation Inhibitory Effects of N-((1H-Pyrazol-1-yl)alkyl)benzoylamides." ARZNEIM. FORSCH., vol. 40, no. 6, 1990, pages 705-709, XP002112557
- E.POMARNACKA ET AL.: "Synthesis and Hypoglycemic Properties of some N-((alkyl-1-pyrazolyl)alkyl)biguanides." ACTA POL. PHARM., vol. 42, no. 3, 1985, pages 236-239, XP002112558
- A.S.POGOSYAN ET AL.: "Alkylation of Azoles by beta-functionality Substituted Alkyl Halides under Phase-Transfer Catalysis Conditions." ZH. PRIKL. KHIM., vol. 59, no. 6, 1985, pages 1296-1300, XP002112559
- DATABASE WPI Section Ch, Week 9240 Derwent Publications Ltd., London, GB; Class B02, AN 92-327578 XP002112560 & JP 04 234891 A (FUJISAWA PHARM CO LTD), 24 août 1992 (1992-08-24)

## Description

La présente invention concerne de nouveaux dérivés de l'érythromycine, leur procédé de préparation et leur application comme médicaments.

La demande EP-A-0596 802 décrit notamment des dérivés 3-oxo 11, 12 carbamate cyclique de l'érythromycine substitués sur l'atome d'azote du carbamate par une chaîne -(CH₂)ₙ-Ar₁, dans laquelle Ar₁ représente un radical hétéroaryl à 5 ou 6 chaînons éventuellement substitué, comportant un ou plusieurs hétéroatomes.

Le document R. Ferroni et al, Arzneim. Forsch., Vol.40, n°6, 1990, p.705-709 - XP 002112557 décrit des composés de type N-((1H-Pyrazol-1-yl)alkyl) benzoylamides ainsi que leurs effets inhibiteurs dans l'aggrégation plaquettaire.

Enfin, la demande EP-A 0799833 décrit notamment des dérivés 3-oxo 11, 12-carbamate cyclique de l'érythromycine substitué sur l'atome d'azote du carbamate par une chaîne -X-Y-Ar, dans laquelle -X-Y- peut représenter un alkylène et Ar un radical hétéroaryle éventuellement substitué.

L'invention a pour objet les composés de formule (I) ainsi que leurs sels d'addition avec les acides : 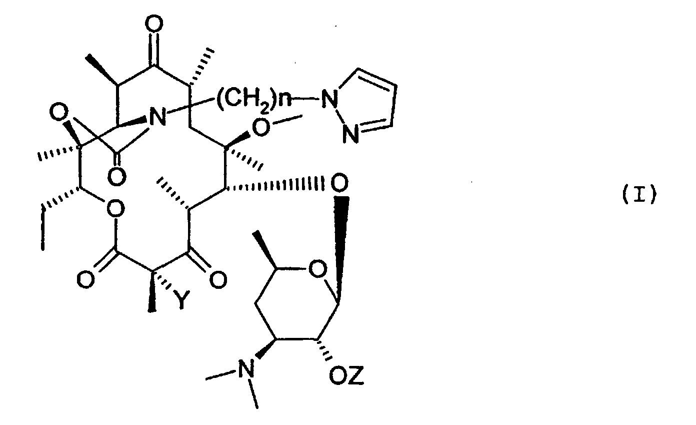 dans laquelle
Y représente un atome d'hydrogène ou un atome de fluor,
n représente un nombre entier compris entre 1 et 8,
Z représente un atome d'hydrogène ou le reste d'un acide carboxylique, substitué sur le pyrazole par un ou plusieurs radicaux aryl renfermant jusqu'à 14 atomes de carbone ou hétéroaryl renfermant un ou plusieurs atomes d'azote, d'oxygène ou de soufre, les radicaux aryl ou hétéroaryl pouvant être eux-mêmes substitués par un ou plusieurs radicaux alkyl, alkényl, alkynyl, O-alkyl, O-alkényl, O-alkynyl, S-alkyl, S-alkényl, S-alkynyl renfermant jusqu'à 8 atomes de carbone, un ou plusieurs radicaux OH, NH₂, C≡N, NO₂, CF₃.

Comme exemple de sels d'addition des présents dérivés avec les acides minéraux ou organiques, on peut citer les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et spécialement les acides stéarique, éthylsuccinique ou laurylsulfonique. Ces sels sont formés avant tout au niveau du groupe diméthylamino mais aussi, le cas échéant, au niveau des autres groupes amino de la molécule.

Dans la définition des substituants, le radical alkyl, alkényl ou alkynyl est de préférence un radical méthyl, éthyl, propyl, isopropyl, n-butyl, isobutyl, terbutyl, décyl ou dodécyl, vinyl, allyl, éthynyl, propynyl, propargyl, cyclobutyl, cyclopentyl ou cyclohexyl.

Le radical aryl peut-être un radical phényl ou naphtyl.

Le radical hétéroaryl substitué ou non peut être le radical thiényl, furyl, pyrolyl, thiazolyl, oxazolyl, imidazolyl, thiadiazolyl, pyrazolyl ou isopyrazolyl, un radical pyridyl, pyrimidyl, pyridazinyl ou pyrazinyl ou encore un radical indolyl, benzofurannyl, benzothiazyl ou quinoléinyl. Ces radicaux aryles peuvent comporter un ou plusieurs substituants choisis parmi les groupements mentionnés ci-dessus.

L'invention a tout spécialement pour objet les composés de formule (I) dans lesquels Z représente un atome d'hydrogène, ceux dans lesquels n représente le nombre 4, Ceux
dans lesquels le radical 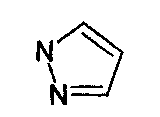 est substitué par un radical

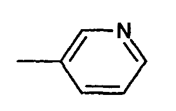

L'invention a tout spécialement pour objet les composés de formule (I) dans lesquels Y représente un atome d'hydrogène.

Parmi les composés préférés, on peut citer tout particulièrement les composés dont la préparation est donnée ci-après dans la partie expérimentale et tout particulièrement le composé de l'exemple 1

Les produits de formule générale (I) possèdent une très bonne activité antibiotique sur les bactéries gram^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aigües primitives ou post grippales, broncho-pneumonie, suppuration pulmonaires, les streptococcies telles que les angines aigües, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, le produit de l'exemple 1 et ses sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou-émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 3000 mg par jour par voie orale, chez l'adulte pour le produit de l'exemple 1.

L'invention a également pour objet un procédé de préparation caractérisé en ce que l'on soumet un composé de formule (II) 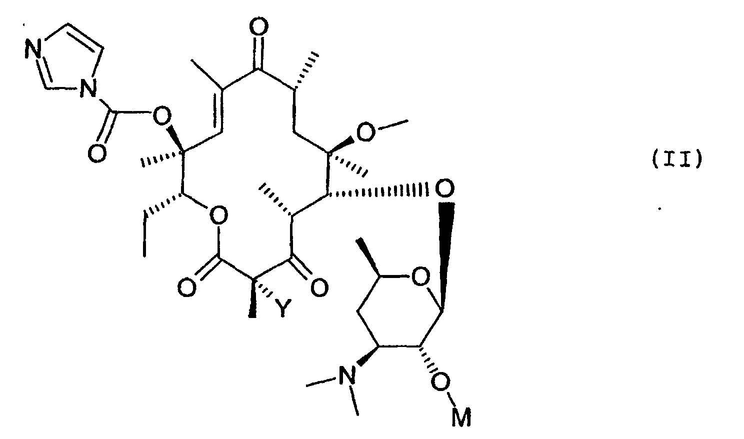 dans laquelle Y conserve sa signification précédente et M représente le reste d'un acide à l'action d'un composé de formule (III) 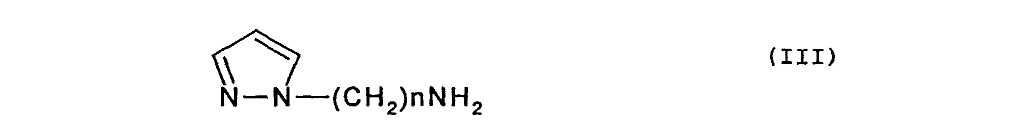 dans lequel le cycle pyrazole est substitué et n est défini comme dans la formule (I) pour obtenir le composé de formule (I) dans lequel Z représente le reste d'un acide, puis si désiré soumet ce composé de formule (I) à l'action d'un agent de libération de l'hydroxyle en 2' pour obtenir le composé de formule (I) correspondant dans lequel Z représente un atome d'hydrogène que l'on soumet si désiré à l'action d'un acide pour en former le sel,
- la réaction du composé de formule (II) avec le composé de formule (III) a lieu au sein d'un solvant tel que par exemple l'acétonitrile, le diméthylformamide ou encore le tétrahydrofuranne, le diméthoxyéthane ou le diméthylsulfoxyde,
- l'hydrolyse de la fonction ester en 2' est réalisée à l'aide du méthanol ou de l'acide chlorhydrique aqueux,
- la salification est réalisée au moyen d'acides selon les procédés classiques.

Les composés de formule (II) dans lesquels Y représente un atome d'hydrogène, utilisés comme produits de départ sont décrits et revendiqués dans la demande de brevet européen 0 596 802.

Les composés de formule (II) dans lesquels Y représente un atome de fluor peuvent être préparés comme indiqué ci-après dans la partie expérimentale.

L'invention a également pour objet à titre de produits chimiques nouveaux, les composés de formule (III) telle que définie plus haut et plus spécialement le composé de formule (III) dont la préparation est donnée ci-après dans la partie expérimentale.

### EXEMPLE 1 : 11,12-didéoxy-3-de [(2,6-didéoxy-3-C-méthyl-3-O-méthyl-.alpha.-L-ribohexopyranosyl)oxy]-6-O-méthyl-3-oxo-12,11-[oxycarbonyl [ [4- [3- (3-pyridinyl)-1H-pyrazol-1-yl]butyl]imino]]-érythromycine

On porte à 55°C pendant 21 heures un mélange de 26 cm³ d'acétonitrile, 2,5 cm³ d'eau, 5,13 g d'amine préparée ci-après (préparation 1) et 6,20 g de 2'-acétate et 12-(1H-imidazol-1-ylcarboxylate) de 10,11-didéhydro-11-déoxy-3-de [(2,6-didéoxy-3-C-méthyl-3-O-méthyl-.alpha.-L-ribohexopyranosyl)oxy]-6-O-méthyl-3-oxo-érythromycine. On verse le mélange réactionnel dans l'eau, extrait à l'acétate d'éthyle et sèche. On obtient 8,02 g de produit qui sont placés dans 70 cm3 de méthanol. On porte le mélange réactionnel au reflux pendant 1 heure 30. On chromatographie le produit obtenu sur silice en éluant avec le mélange chlorure de méthylène, méthanol, ammoniaque 95-5-0,5. On obtient 3,59 g de produit fondant à 143-145°C. RMN CDCl₃ ppm

| **Numéro** | ^{**1**}**H** | **Numéro** | ^{**1**}**H** |
|---|---|---|---|
| 1 | | 1' | 4,28 |
| 2 | 3,86 | 2' | 3,16 |
| 3 | | 3' | 2,44 |
| 4 | 3,07 | 4' | 1,67 - 1,23 |
| 5 | 4,21 | 5' | 3,52 |
| 6 | | 5' Me | 1,24 |
| 7 | 1,59 - 1,82 | N(Me)₂ | 2,26 |
| 8 | 2,60 | NCH2 | 3,60 à 3,80 |
| 9 | | CH2 | 1,65 |
| 10 | 3,13 | CH2 | 1,95 |
| 11 | 3,57 | CH2N | 4,21 |
| 12 | | Pyrazole H4 | 6,55 |
| 13 | 4,94 | Pyrazole H5 | 7,47 |
| 14 | 1,95 - 1,57 | pyridine | |
| 15 | 0,85 | H2 | 9,00 |
| 2Me | 1,36 | H4 | 8,11 |
| 4Me | 1,31 | H5 | 7,31 |
| 6Me | 1,33 ou 1,47 | H6 | 8,51 |
| 8Me | 1,16 | | |
| 10Me | 1,00 | | |
| 12Me | 1,33 ou 1,47 | | |
| 60Me | 2,60 | | |

### Préparation 1 : 3-(3-pyridinyl)-1H-pyrazole-1-butanamine

### Stade A : 2-[4-[3-(3-pyridinyl)-1H-pyrazol-1-yl]butyl]-1H-isoindole-1,3(2H)-dione

On introduit goutte à goutte en 1 heure en maintenant la température ci-dessous de 30°C ou égale à 30°C, 15,45 g de 3-(1H pyrazol-3-yl)-pyridine préparée comme indiqué dans CA 68 P 95812 g (1968) dans un mélange de 20 ml de DMF et 6,13 g d'hydrure de sodium. On ajoute goutte à goutte une solution de 29,90 g de 2-(4-bromobutyl)-1H-isoindole-1,3(2H)-dione et 110 ml de DMF. On agite 30 minutes à la température ambiante. On concentre, verse sur 300 ml d'eau refroidie à 10°C, extrait à l'acétate d'éthyle, lave à l'eau, sèche filtre et concentre. On reprend au chlorure de méthylène, sèche, filtre et concentre. On obtient 35,87 d'un produit que l'on cristallise dans l'éther éthylique, essore, lave à l'eau, sèche. On obtient 22,93 g de produit recherché.

### Stade B : 3-(3-pyridinyl)-1H-pyrazole-1-butanamine

On ajoute 7 ml d'hydrate d'hydrazine dans une suspension renfermant 450 ml d'éthanol et 22,33 g de produit du stade A. On porte au reflux pendant 15 heures. On évapore l'éthanol, agite le mélange réactionnel avec 200 ml d'acétate d'éthyle, lave à l'eau salée, sèche, filtre et concentre. On obtient ainsi 9,60 g de produit recherché.

### EXEMPLE 2 : 11,12-didéoxy-3-de [(2,6-didéoxy-3-C-méthyl-3-O-méthyl-.alpha.-L-ribohexopyranosyl)oxy]2-fluoro-6-O-méthyl-3-oxo-12,11-[oxycarbonyl[[4-[3-(3-pyridinyl)-1H-pyrazol-1-yl]butyl]imino]]-érythromycine

En opérant comme précédemment, à partir du dérivé de formule (II) 2 fluoré correspondant préparé comme indiqué ci-après, on a obtenu le produit recherché F = 117 ^{~}121°C. Préparation du composé de formule (II) dans lequel Y représente un atome de fluor.

### 2'-acétoxy 2 α-fluoro de 12-(oxycarbonylimidazol)11-déoxy 10,11-didéhydro 3- de [(2,6-didéoxy 3-C-méthyl 3-0-méthyl α -L-ribohexopyranosyl)oxy] 6-0-méthyl 3-oxo érythromycine.

### Stade A : 11-déoxy 10,11-didéhydro 3-de[(2,6-didéoxy 3-0-méthyl α-L-ribohexopyranosyl) oxy]6-0-méthyl 3-oxo érythromycine.

On agite pendant 44 heures un mélange de 8,722 g de 2'-acétate de 11-déoxy 10,11-didéhydro 3-de[(2,6-didéoxy 3-0-méthyl α L-ribohexopyranosyl)oxy]6-0-méthyl 3-oxo érythromycine et 350 ml de méthanol anhydre (EP 596802). On obtient 8,794 g du produit recherché.

### Stade B : 2'-triméthylsilyloxy de 11-déoxy 10,11-didéhydro 3-de [(2,6-didéoxy 3-0-méthyl α-L-ribohexopyranosyl)oxy] 6-0-méthyl 3-oxo érythromycine.

On agite à température ambiante pendant 4 jours un mélange renfermant 3,08 g du produit du stade précédent, 340 mg d'imidazole, 32 ml de THF anhydride et 1,06 ml d'hexaméthyldisilazane. On évapore à sec, reprend avec un mélange de 60 ml de chlorure de méthylène et de 60 ml de phosphate acide de sodium 0,5 M. On maintient le mélange réactionnel sous agitation pendant 15 minutes, décante, extrait au chlorure de méthylène, sèche et évapore à sec. On obtient 3,345 g du produit recherché.

### Stade C : 2'-triméthylsilyloxy 2α-fluoro de 11-déoxy 10,11-didéhydro 3- de [(2,6-didéoxy 3-0-méthyl α-L-ribohexopyranosyl)oxy] 6-0-méthyl 3-oxo érythromycine.

On ajoute à - 12°C sous atmosphère d'argon 1,24 ml d'une solution de terbutylate de potassium dans le THF 0,97M dans une solution renfermant 668 mg de 2'-triméthylsilyloxy de 11-déoxy 10,11-didéhydro 3-de [(2,6-didéoxy 3-0-méthyl α-L-ribohexopyranosyl)oxy]6-0-méthyl 3-oxo érythromycine et 6,7 ml de THF anhydre. On agite 5 minutes et ajoute 378 mg de N-fluoro dibenzènesulfonimide. On agite 10 minutes à -12°C et laisse revenir à la température ambiante pendant 1 heure 30 minutes. On effectue les opérations d'isolation et purification et obtient 695 mg du produit recherché.

### Stade D : 2α-fluoro de 11-déoxy 10,11-didéhydro 3- de [(2,6-didéoxy 3-0-méthyl 3-0-méthyl α-L-ribohexopyranosyl)oxy] 6-0-méthyl 3-oxo érythromycine

On agite pendant 3 heures 30 minutes un mélange de 5,476 g de produit de l'exemple 2, 50 ml de THF et 11,2 ml de fluorure de tétrabutylammonium 1M dans le THF. On évapore le solvant et ajoute 37 ml d'acétate d'éthyle, 37 ml d'eau et 7,5 ml d'ammoniaque à 20 %. On agite 10 minutes, décante, extrait à l'acétate d'éthyle, sèche, filtre et concentre à sec le filtrat. On chromatographie le produit obtenu sur silice en éluant avec le mélange CH₂CL₂-MeOH ammoniaqué 99-1, puis 98-2, 97-3, 96-4, 95-5. On obtient 2,452 g de produit recherché.

### Stade E : 2'-acétoxy 2α-fluoro de 11-déoxy 10,11-didéhydro 3- de [(2,6-didéoxy 3-0-méthyl α-L-ribohexopyranosyl)oxy] 6-0-méthyl 3-oxo érythromycine

On maintient sous agitation pendant 3 heures 1,02 g de produit du stade A, 10 ml de chlorure de méthylène et 241 µl d'anhydride acétique. On évapore et ajoute 10 ml d'eau et 10 ml d'acétate d'éthyle. On laisse 1 heure à la température ambiante sous agitation, décante, sèche et évapore. On obtient 1,01 g de produit recherché.

### Stade F : 2'-acétoxy 2α-fluoro de 12-(oxycarbonylimidazol) 11-déoxy 10,11-didéhydro 3- de [(2,6-didéoxy 3-C-méthyl-3-0-méthyl α-L-ribohexopyranosyl)oxy] 6-0-méthyl 3-oxo érythromycine

On ajoute à 0°C 0,388 g de carbonyldiimidazole et 24 µL de DBU dans une solution renfermant 1,01 g du produit du stade précédent et 10 ml de THF anhydre. On évapore le THF et ajoute 10 ml d'eau et 10 ml d'acétate d'éthyle. On maintient le mélange réactionnel sous agitation pendant 10 minutes, extrait, sèche et évapore. On obtient 0,902 g de produit recherché brut que l'on chromatographie en éluant avec un mélange acétate d'éthyle-triéthylamine 96-4. On obtient 0,573 g de produit recherché.

### EXEMPLE 3

En opérant comme à l'exemple 1 en utilisant la 4-(3-pyridinyl)-1H-pyrazole-1-butanamine, on a obtenu la 11,12-didéoxy-3-de [(2,6-didéoxy-3-C-méthyl-3-O-méthyl-.alpha.-L-ribohexopyranosyl)oxy]-6-O-méthyl-3-oxo-12,11-[oxycarbonyl[[4-[4-(3-pyridinyl)-1H-pyrazol-1-yl]butyl]-imino]]-érythromycine.
Spectre de masse MH⁺=812⁺
Spectre RMN (300MHz dans CDCl₃)
H2 : 3.84 ppm ; H4 : 3006 ppm ; H5 : 4.22 ppm ; H7 : 1.58 1.83 ppm ; H8 : 2.58 ppm ; H10 : 3.12 ppm ; H11 : 3.56 ppm ; H13 : 4.92 ppm ; H14 : 1.55 1.94 ; H15 : 0.81 ppm ; 2Me : 1.35 ppm ; 4Me 1.29 ppm ; 6 Me : 1.32 ou 1.46 ppm ; 8 Me 1.16 ppm ; 10 Me : 1.01 ppm ; 12 Me : 1.32 ou 1.46 ppm ; 60 Me : 2.6 ppm ; 1' : 4.27 ppm ; 2' : 3.17 ppm ; 3' 2.44 ppm ; 4' : 1.67 et 1.24 ppm ; 5' : 3.55 ppm ; NMe₂ : 2.26 ppm ; NCH₂ : 3.69 ppm ; CH₂ : 1.64 1.94 ppm ; CH₂N : 4.19 ppm ; pyrazole : 7.77 ppm ; pyridine : 8.76 7.75 7.27 8.44 ppm.
Spectre de masse
812⁺ : MH⁺
850⁺ : MK⁺

L'amine de départ a été préparée comme dans la préparation 1 à partir du produit préparé comme indiqué dans le schéma suivant.

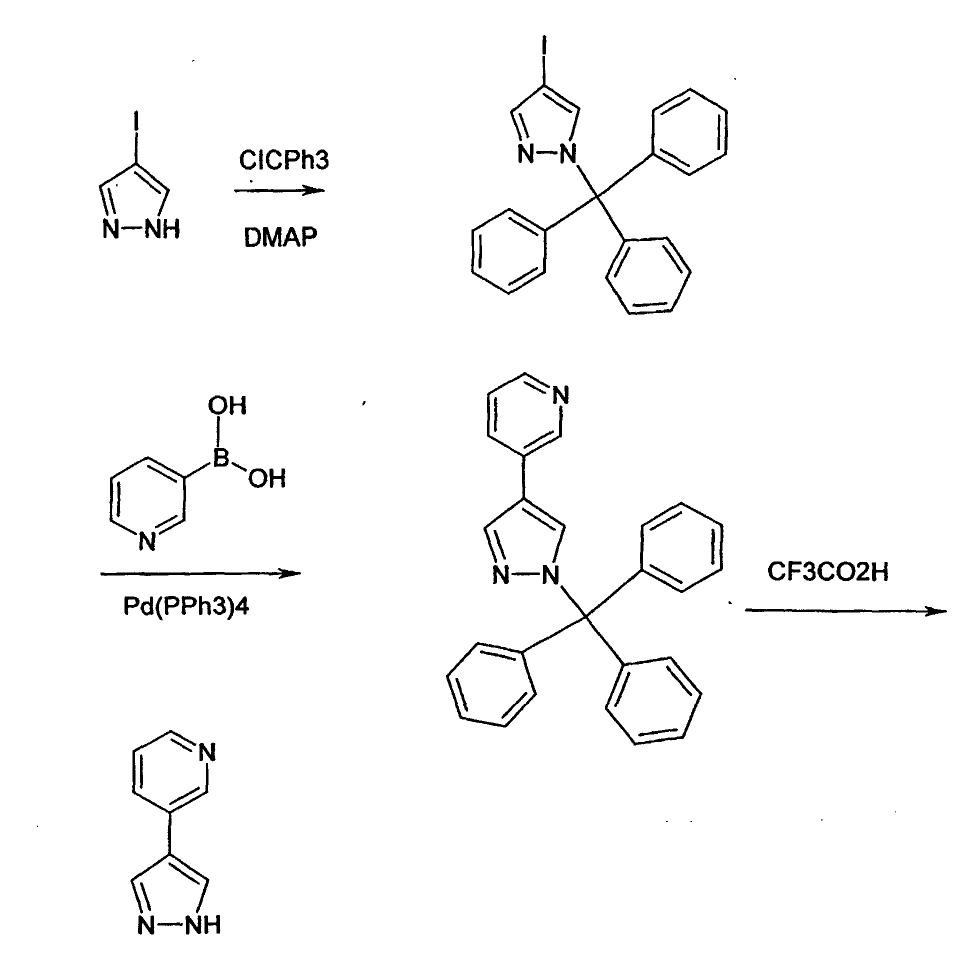

### EXEMPLE 4 : 11,12-didéoxy-3-de [(2,6-didéoxy-3-C-méthyl-3-O-méthyl-.alpha.-L-ribohexopyranosyl)oxy]-2-fluoro-6-O-méthyl-3-oxo-12,11-[oxycarbonyl[[4-[4-(3-pyridinyl)-1H-pyrazol-1-yl]butyl]-amino]]-érythromycine

Spectre RMN (300 MHz dans CDCl3)
H4 : 3.53 ppm ; H5 : 4.05 ppm ; H7 : 1.51 1.88 ppm ; H8 : 2.61 ppm ; H10 : 3.10 ppm ; H11 : 3.42 ppm ; H13 : 4.86 ppm H14 : 1.63 1.95 ; H15 : 0.85 ppm ; 2Me : 1.75 ppm ; 4Me : 1.29 ppm ; 6 Me : 1.31 ou 1.49 ppm ; 8Me : 1.17 ppm ; 10Me : 1.00 ppm ; 12Me : 1.31 ou 1.49 ppm ; 60Me : 2.51 ppm ; 1' : 4.30 ppm ; 2' : 3.19 ppm ; 3' : 2.48 ppm ; 4' : 1.68 et 1.26 ppm ; 5' : 3.53 ppm ; 5'Me : 1.24 ppm ; NMe₂ : 2.28 ppm ; NCH₂ : 3.55 à 3.80 ppm ; CH₂ : 1.61 1.93 ppm ; CH₂N : 4.19 ppm ; pyrazole : 7.75 7.78 ppm ; pyridine : 8.77 7.77 7.27 8.44 ppm
Spectre de masse
830⁺ : MH⁺
158⁺ : Désosamine
673⁺ : 830⁺ -158⁺H

### EXEMPLES DE COMPOSITION PHARMACEUTIQUE

On a préparé des comprimés renfermant :

| | |
|---|---|
| Produit de l'exemple 1 | 150 mg |
| Excipient q.s.p. | 1 g |

Détail de l'excipient : amidon, talc, stéarate de magnésium

### ETUDE PHARMACOLOGIQUE DES PRODUITS DE L'INVENTION

### A - Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de 24 heures à l'étuve à 37 °C, l'inhibition de la croissance est appréciée par transillumination, ce qui permet de déterminer les concentrations minimales inhibitrices (C.M.I.) exprimées en microgrammes/cm³. Les résultats suivants ont été obtenus avec le produit de l'exemple 1 : (lecture après 24 heures).

| Souches bactériennes à GRAM⁺ | | |
|---|---|---|
| S.aureus | 011UC4 | 0.040 |
| S.aureus | 011UC4+sérum 50% | 0.600 |
| S.aureus | 011B18c | |
| S.aureus | 011GR12c | |
| S.aureus | 011GO25i | 0.600 |
| S.epidermidis | 012GO11i | 0.040 |
| S.aureus | 011CB20c | |
| S.epidermidis | 012GO40c | |
| S. pyogenes | 02A1UC1 | 0.02 |
| S. agalactiae | 02B1HT1 | 0.02 |
| S. faecalis | 02D2UC1 | 0.02 |
| S. faecium | 02D3HT1 | 0.02 |
| Streptococcus gr.G | 02GOGR5 | 0.02 |
| S. mitis | 02MitCB1 | 0.02 |
| S. agalactiae | 02B1SJ1c | 0.050 |
| S. faecalis | 02D2DU15c | 5.000 |
| Streptococcus gr.G | 02Gogr4c | |
| S. sanguis | 02SGr10i | 0.02 |
| S. mitis | 02MitGR16i | 0.02 |
| S. pneumoniae | 032UC1 | 0.02 |
| S. pneumoniae | 030GR20 | 0.02 |
| S. pneumoniae | 030SJ5i | 0.040 |
| S. pneumoniae | 030CR18c | 0.300 |
| S. pneumoniae | 030PW23c | 0.02 |
| S. pneumoniae | 030RO1i | 0.150 |
| S. pneumoniae | 030SJ1c | 0.150 |

De plus, le produit de l'exemple 1 a notamment une activité intéressante sur les souches bactériennes à gram⁻ Haemophilus Influenzae 351HT3, 351CB12, 351CA1 et 351GR6.

## Revendications

1. Les composés de formule (I) ainsi que leurs sels d'addition avec les acides : 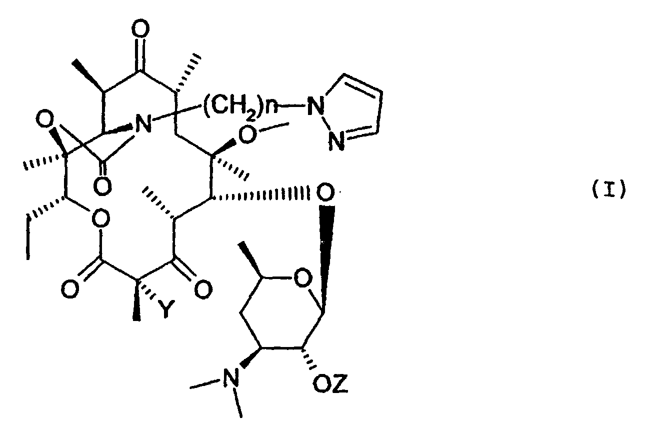 dans laquelle
Y représente un atome d'hydrogène ou un atome de fluor,
n représente un nombre entier compris entre 1 et 8,
Z représente un atome d'hydrogène ou le reste d'un acide carboxylique, substitué sur le pyrazole par un ou plusieurs radicaux aryl renfermant jusqu'à 14 atomes de carbone ou hétéroaryl renfermant un ou plusieurs atomes d'azote, d'oxygène ou de soufre, les radicaux aryl ou hétéroaryl pouvant être eux-mêmes substitués par un ou plusieurs radicaux alkyl, alkényl, alkynyl, O-alkyle, O-alkényle, O-alkynyl, S-alkyl, S-alkényl, S-alkynyl renfermant jusqu'à 8 atomes de carbone, un ou plusieurs radicaux OH, NH₂, C≡N, NO₂, CF₃.

2. Les composés de formule (I) définis à la revendication 1 dans lesquels Z représente un atome d'hydrogène.

3. Les composés de formule (I) définis à la revendication 1 ou 2 dans lesquels n représente le nombre 4.

4. Les composés de formule définis à la revendication 1, 2 ou 3 dans lesquels le radical 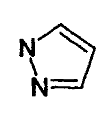 est substitué par un radical

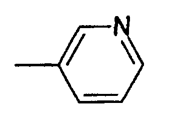

5. Le composé de formule (I) défini à la revendication 1 dans laquelle Y représente un atome d'hydrogène.

6. Le composé de formule répondant à la formule (I) de la revendication 1 suivant : 11,12-didéoxy-3-de [(2,6-didéoxy-3-C-méthyl-3-O-méthyl-.alpha.-L-ribohexopyranosyl)oxy]-6-O-méthyl-3-oxo-12,11-[oxycarbonyl[[4-[3-(3-pyridinyl)-1H-pyrazol-1-yl]butyl]imino]]-érythromycine.

7. A titre de médicaments les composés de formule (I) définis à l'une quelconque des revendications 1 à 5 ainsi que leurs sels d'addition avec les acides pharmaceutiquement acceptables.

8. A titre de médicaments, le composé de formule (I) défini à la revendication 6 ainsi que ses sels d'addition avec les acides pharmaceutiquement acceptables.

9. Les compositions pharmaceutiques renfermant comme principe actif au moins un médicament selon la revendication 7 ou 8.

10. Procédé de préparation des composés de formule (I) définis à l'une quelconque des revendications 1 à 6 **caractérisé en ce que** l'on soumet un composé de formule (II) 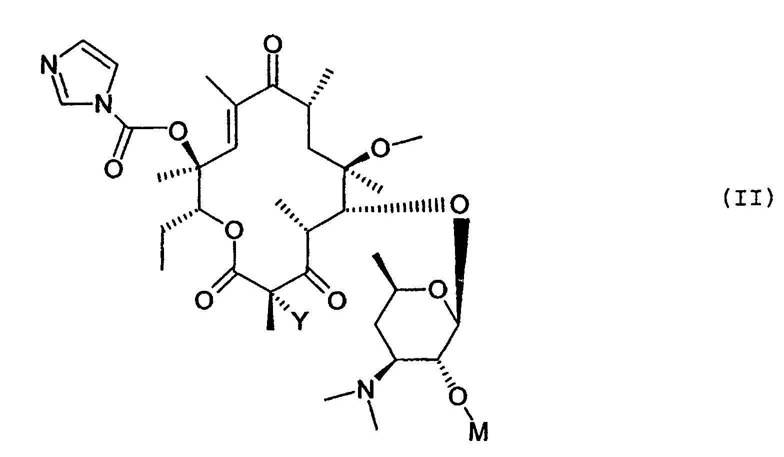 dans laquelle Y conserve sa signification précédente et M représente le reste d'un acide à l'action d'un composé de formule (III) 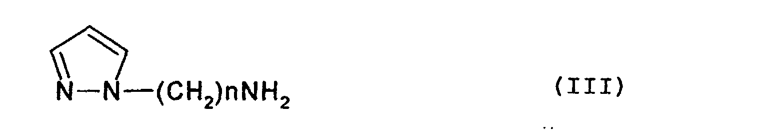 dans lequel le cycle pyrazole est substitué et n est défini comme indiqué à la revendication 1, pour obtenir le composé de formule (I) dans lequel Z représente le reste d'un acide, puis si désiré soumet ce composé de formule (I) à l'action d'un agent de libération de l'hydroxyle en 2' pour obtenir le composé de formule (I) correspondant dans lequel Z représente un atome d'hydrogène que l'on soumet si désiré à l'action d'un acide pour en former le sel.

11. A titre de produits chimiques les composés de formule (III) définis à la revendication 10.

12. A titre de produit chimique défini à la revendication 11, les composés de formule (III) suivants : 3-(3-pyridinyl)-1H-pyrazole-1-butanamine, 4-(3-pyridinyl)-1H-pyrazole-1-butanamine.

## Patentansprüche

1. Verbindung der Formel (I) sowie ihre Additionssalze mit Säuren 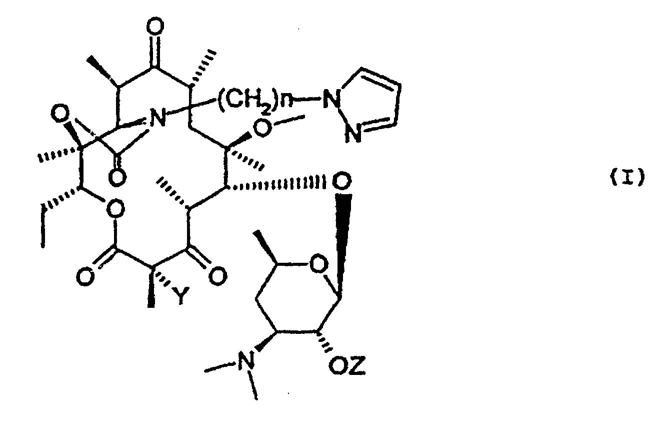 worin
Y ein Wasserstoffatom oder ein Fluoratom darstellt;
n eine ganze Zahl zwischen 1 und 8 darstellt;
Z ein Wasserstoffatom oder den Rest einer Carbonsäure darstellt,
die am Pyrazol durch eine oder mehrere ArylGruppen, die bis zu 14 Kohlenstoffatome umfassen, oder Heteroaryl-Gruppen, die ein oder mehrere Stickstoff-, Sauerstoff- oder Schwefelatome umfassen, substituiert ist, wobei die Aryl- und Heteroaryl-Gruppen selbst durch eine oder mehrere Alkyl-, Alkenyl-, Alkinyl-, 0-Alkyl-, O-Alkenyl-, O-Alkinyl-, S-Alkyl-, S-Alkenyl-, S-Alkinyl-Gruppen, die bis zu 8 Kohlenstoffatome umfassen, eine oder mehrere der Gruppen OH, NH₂, C≡N, NO₂, CF₃ substituiert sein können.

2. Verbindungen der Formel (I), die in Anspruch 1 definiert sind, wobei Z ein Wasserstoffatom darstellt.

3. Verbindungen der Formel (I), die in Anspruch 1 oder Anspruch 2 definiert sind, wobei n die Zahl 4 darstellt.

4. Verbindungen der Formel (I), die in Anspruch 1, 2 oder 3 definiert sind, in denen die Gruppe 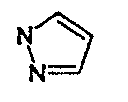 durch eine Gruppe 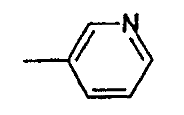 substituiert ist.

5. Verbindung der Formel (I), die in Anspruch 1 definiert ist, wobei Y ein Wasserstoffatom darstellt.

6. Verbindung der Formel (I) nach Anspruch 1 mit dem folgenden Namen: 11,12-Didesoxy-3-de[(2,6-didesoxy-3-Cmethyl-3-O-methyl-alpha-L-ribohexapyranosyl)oxy]-6-0-methyl-3-oxo-12,11-[oxycarbonyl[[4-[3-(3-pyridinyl)-1Hpyrazol-1-yl]butyl]imino]]-erythromycin.

7. Verbindungen der Formel (I), die in einem der Ansprüche 1 bis 5 definiert sind, sowie ihre Additionssalze mit pharmazeutisch annehmbaren Säuren als Arzneimittel.

8. Verbindung der Formel (I), die in Anspruch 6 definiert ist, sowie die Additionssalze mit pharmazeutisch annehmbaren Säuren als Arzneimittel.

9. Pharmazeutische Zusammensetzungen, die als Wirkstoff mindestens ein Arzneimittel nach Anspruch 7 oder 8 umfassen.

10. Verfahren zur Herstellung der Verbindungen der Formel (I), die in einem der Ansprüche 1 bis 6 definiert sind, **dadurch gekennzeichnet, daß** man eine Verbindung der Formel (II): 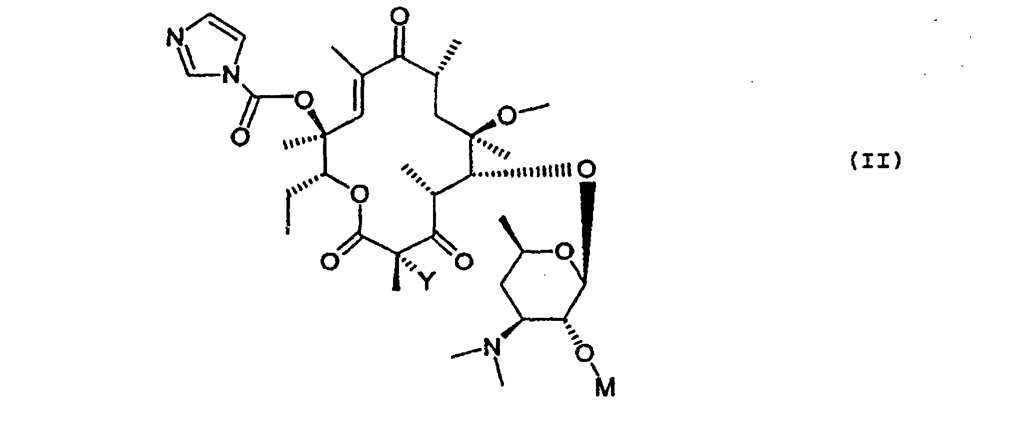 in der Y seine vorstehende Bedeutung behält und M den Rest einer Säure darstellt, der Umsetzung mit einer Verbindung der Formel (III) : 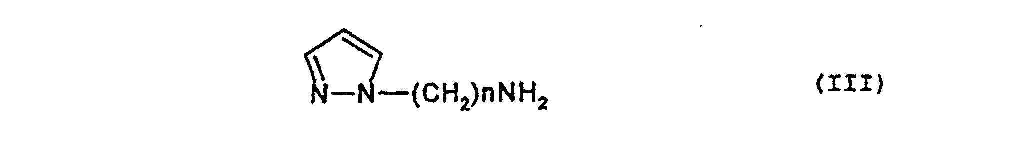 in der der Pyrazol-Ring so substituiert und n so definiert ist, wie es in Anspruch 1 angegeben ist, unterwirft, um die Verbindung der Formel (I) zu erhalten, in der Z den Rest einer Säure darstellt; man dann gewünschtenfalls diese Verbindung der Formel (I) der Einwirkung eines Agenzes zur Freisetzung des Hydroxyls in 2' unterwirft, um die entsprechende Verbindung der Formel (I) zu erhalten, in der Z ein Wasserstoffatom darstellt, die man auf Wunsch zur Bildung des Salzes der Wirkung einer Säure unterwirft.

11. Verbindungen der Formel (III), die in Anspruch 10 definiert sind, als chemische Produkte.

12. Folgende Verbindungen der Formel (III): 3-(3-Pyridinyl)-lH-pyrazol-1-butanamin, 4-(3-Pyridinyl)-1H-pyrazol-1-butanamin, als chemisches Produkt, das in Anspruch 11 definiert ist.

## Claims

1. The compounds of formula (I) and their addition salts with acids: 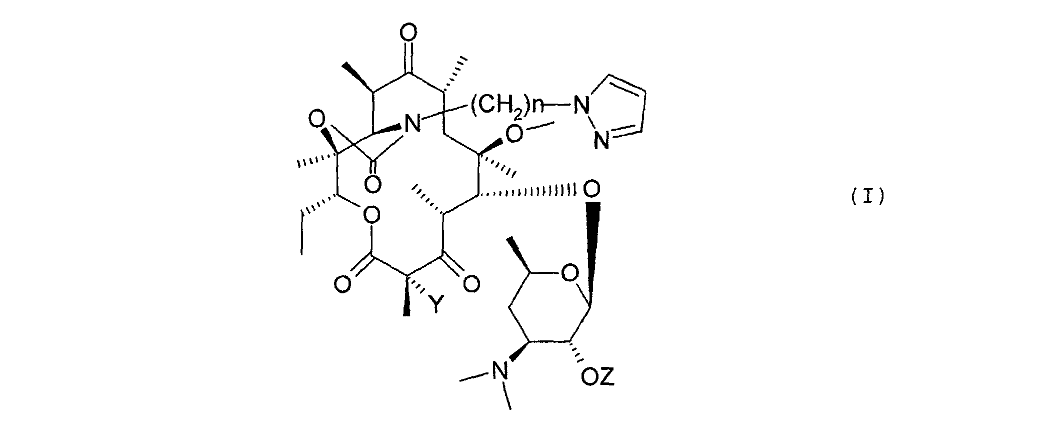 in which
Y represents a hydrogen atom or a fluorine atom,
n represents an integer comprised between 1 and 8,
Z represents a hydrogen atom or the remainder of a carboxylic acid, substituted on the pyrazole by one or more aryl radicals containing up to 14 carbon atoms or heteroaryl radicals containing one or more nitrogen, oxygen or sulphur atoms, the aryl or heteroaryl radicals themselves being able to be substituted by one or more alkyl, alkenyl, alkynyl, O-alkyl, O-alkenyl, O-alkynyl, S-alkyl, S-alkenyl, S-alkynyl containing up to 8 carbon atoms, one or more OH, NH₂, C≡N, NO₂, CF₃ radicals.

2. The compounds of formula (I) as defined in claim 1 in which Z represents a hydrogen atom.

3. The compounds of formula (I) defined in claim 1 or 2 in which n represents the number 4.

4. The compounds of formula defined in claim 1, 2 or 3 in which the 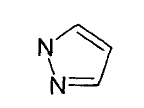 radical is substituted by a 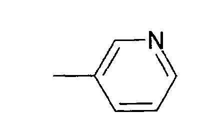 radical

5. The compound of formula (I) defined in claim 1 in which Y represents a hydrogen atom.

6. The following compound of formula corresponding to formula (I) of claim 1: 11,12-dideoxy-3-de [(2,6-dideoxy-3-C-methyl-3-O-methyl-.alpha.-L-ribohexopyranosyl)oxy]-6-O-methyl-3-oxo-12,11-[oxycarbonyl[[4-[3-(3-pyridinyl)-1H-pyrazol-1-yl]butyl]imino]]-erythromycin.

7. As medicaments, the compounds of formula (I) defined in any one of claims 1 to 5 as well as their addition salts with pharmaceutically acceptable acids.

8. As medicaments, the compound of formula (I) defined in claim 6 as well as its addition salts with pharmaceutically acceptable acids.

9. The pharmaceutical compositions containing as active ingredient at least one medicament according to claim 7 or 8.

10. Process for the preparation of the compounds of formula (I) defined in any one of claims 1 to 6 **characterized in that** a compound of formula (II) 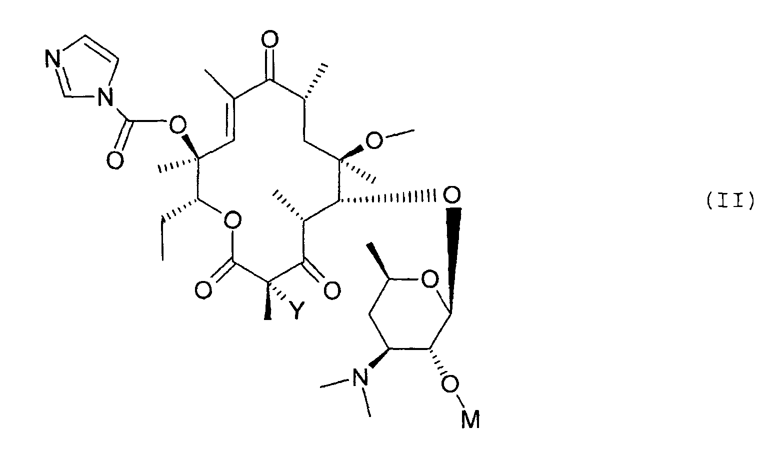 in which Y retains its previous meaning and M represents the remainder of an acid is subjected to the action of a compound of formula (III) 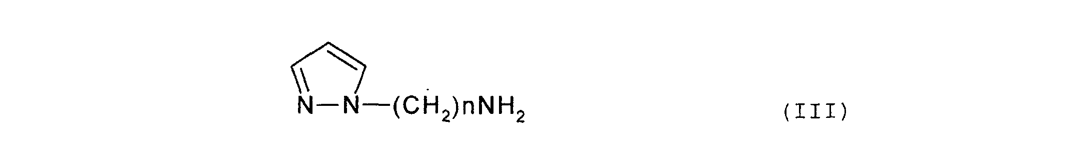 in which the pyrazole ring is substituted and n is defined as indicated in claim 1, in order to obtain the compound of formula (I) in which Z represents the remainder of an acid, then, if desired, this compound of formula (I) is subjected to the action of an agent which releases the hydroxyl in position 2' in order to obtain the corresponding compound of formula (I) in which Z represents a hydrogen atom which if desired is subjected to the action of an acid in order to form the salt.

11. As chemical products, the compounds of formula (III) defined in claim 10.

12. As a chemical product defined in claim 11, the following compounds of formula (III): 3-(3-pyridinyl)-lH-pyrazole-1-butanamine, 4-(3-pyridinyl)-1H-pyrazole-1-butanamine.
